# EUROPEAN PATENT SPECIFICATION

(11) **EP 2 367 552 B1**
(45) Date of publication and mention of the grant of the patent: **20.07.2016**
(21) Application number: 09832240.7
(22) Date of filing: 10.12.2009
(51) Int. Cl.: A61K 31/44, A61K 9/14, A01N 59/20

(54) **SILICA-BASED ANTIBACTERIAL AND ANTIFUNGAL NANOFORMULATION**
ANTIBAKTERIELLE UND ANTIMYKOTISCHE NANOFORMULIERUNG AUF KIESELERDEBASIS
NANO-FORMULATION ANTIBACTÉRIENNE ET ANTIFONGIQUE À BASE DE SILICE

(30) Priority: 10.12.2008 US 332021
(43) Date of publication of application: 28.09.2011
(73) Proprietor: University Of Central Florida Research Foundation, Inc., Orlando, FL 32826 (US); Santra, Swadeshmukul, Orlando, FL 32825 (US)
(72) Inventor: SANTRA, Swadeshmukul, Orlando, FL 32825 (US)
(74) Representative: REHBERG HÜPPE + PARTNER
(86) International application number: PCT/US2009/006496
(87) International publication number: WO 2010/068275

(56) References cited:
- US-A1- 2004 096 421
- US-A1- 2007 104 751
- US-A1- 2007 140 951
- US-A1- 2007 269 382
- US-B1- 6 471 976

## Description

### FIELD OF THE INVENTION

This invention relates to silica-based nanoformulations, and in particular to, compositions and methods for fabricating silica-based nanoparticles and silica-based nanogels, or a combination thereof, that provide a unique nanoenvironment to host antibacterial and antifungal agents, method of synthesis and use.

### BACKGROUND AND PRIOR ART

### Global Concern

The globalization of business, travel and communication brings increased attention to worldwide exchanges between communities and countries, including the potential globalization of the bacterial and pathogenic ecosystem. Bactericides and fungicides have been developed to control diseases in man, animal and plants, and must evolve to remain effective as more and more antibiotic, pesticide and insecticide resistant bacteria and fungi appear around the globe.

Bacterial resistance to antimicrobial agents has also emerged, throughout the world, as one of the major threats to both man and the agrarian lifestyle. Resistance to antibacterial and antifungal agents has emerged as an agricultural issue that requires attention and improvements in the treatment materials in use today.

For example, focusing on plants, there are over 300,000 diseases that afflict plants worldwide, resulting in billions of dollars of annual crop losses. In 1990, over 7.3 billion dollars were spent in the United States on pesticide products.

Antibacterial and antifungal (Anti-B/F) agents include, but are not limited to, metallic copper (Cu), copper salts, copper complexes, metallic zinc (Zn), zinc oxides, zinc salts, metallic silver (Ag), silver salts, silver complexes, titanium dioxide (TiO₂), cerium oxides, magnesium oxides, zirconium oxides, polyethyleneimine (PEI), carbon, mixed carbon or soot, fullerenes, carbon nanotubes and the like. The preceding compounds are only a few examples of antibacterial and antifungal agents used by man in a quest to control or eliminate infectious diseases in our global environment.

The antibacterial/antifungal (Anti-B/F) formulations in existence today could be improved and made more effective if the following features were included in the formulation. For example, a desirable formulation would have an increased, uniform distribution on a treated surface area, improved adherence to treated surface, a means for controlling and sustaining the release of the active ingredients, and dosage levels that avoid any toxic impact on the environment and/or the treated surface.

The present invention provides a composition that hosts antibacterial/antifungal formulations and together, the host composition and antibacterial/antifungal ingredients provide functional benefits that solve many problems and overcome many limitations in the prior art.

A synthesis method for preparation of a silica matrix with embedded metallic particles is reported in U.S. Patent 6,548,264 to Tan et al., U.S. 6,924,116 to Tan et al., and U.S. Patent 7,332,351 to Tan et al.

### Use of Copper (Cu) Fungicides/Bactericides

In modem agriculture, copper (Cu) compounds are widely used as fungicides /bactericides. Cu compounds, in relatively low concentration, are quite toxic to thalophyte organisms, such as, fungi, bacteria, and algae. This property of toxicity has been utilized for over 100 years for control of fungal and bacterial diseases of plants. In 1761, it was discovered that seed grains soaked in a weak solution of Cu sulfate inhibited seed-borne fungi.

The greatest breakthrough for Cu salts undoubtedly came when the French scientist Millardet announced to the world in 1885 that he had found a cure for the dreaded mildew using mixtures of Cu sulfate, lime and water (known as Bordeaux mixture). Cu based fungicides/bactericides are used worldwide because Cu compounds are relatively safe; development of resistance by plant pathogens has been minimal; in demand by developing third world countries and the increasing requirements for food requiring more efficient agriculture; and an increase in government regulations and restrictions or outright banning of alternative products due to their toxicological and environmental impact requires safe treatment formulations, as discussed by H.W. Richardson in Handbook of Copper Compounds and Applications, "Copper fungicides/bactericides" H. W. Richardson, Editor, 1997, Marcel Dekker, Inc.: New York, NY, pages 93-122.

The toxicity of Cu is largely due to its tendency to alternate between its cuprous, Cu(I), and cupric, Cu(II), oxidation states. Under aerobic conditions, this redox cycling leads to the generation of highly reactive hydroxyl radicals that readily and efficiently damage biomolecules, such as DNA, proteins, and lipids. The underlying Fenton-like reactions involving reactive oxygen species can be described as Cu-catalyzed Haber-Weiss reactions. While the reaction of dihydrogen peroxide with superoxide primarily has a negligible rate constant as shown below in equation 1, this rate is greatly accelerated in the presence of Cu.

**H₂O₂ + O₂⁻ → O₂ + OH⁻ + OH*** **(1)**

Cu ions are believed to catalyze this reaction as shown in equations 2 and 3.

Cu(II) is initially reduced by superoxide as shown in equation 2,

**Cu(II) + O₂⁻ → Cu(I) + O₂** **(2)**

followed by reoxidation by dihydrogen peroxide as shown in equation 3,

**Cu(I) + H₂O₂ → Cu(II) + O₂ + OH⁻ + OH^{*}** **(3)**

resulting in a net production of the hydroxyl radical.

Most fungicides and bactericides do little to kill an established infection of fungi and bacteria, respectively, but act by protecting the host from the possibility of infection. If the protectant is applied after the onset of disease symptoms, control will often be minimal. Similarly, Cu compounds inhibit germination of the fungal spore or bacterial cell, the primary "seeds" responsible for dissemination and reproduction of the fungus or bacterium. Because the spore or cell removed from the current infection cycle does not mature nor reproduce in the presence of Cu, the fungus or bacterium are effectively killed.

The fungicidal/bactericidal value is a measure of the toxicity of a material to the pathogen and is usually expressed as an LD₅₀, primarily a laboratory based *in vitro* toxicity measurement, wherein LD stands for Lethal Dose and LD₅₀ is the amount of material or dose which causes the death of 50% of the target population.

The protective value is a measure of the ability of a material to protect the host organism, for example, a plant, from infection, which is primarily a measurement under field conditions. For example, Cu sulfate has an excellent ability to inhibit fungal spore or bacterial cell germination in the laboratory; however, in the field it exhibits no persistence, because of its solubility. It has limited protective value because it is quickly removed from the plant surface at the first rain. Furthermore, Cu sulfate may produce sufficient soluble Cu to be toxic to the plant (phytotoxic). A Cu compound must be chosen that is relatively resistant to weathering and supplies enough Cu to be toxic to the fungal spores and bacterial cells without adversely affecting the plant.

An important consideration is whether to use "soluble" or "insoluble" copper (Cu) for long-term fungicidal or bactericidal protection. The "soluble" Cu refers to Cu based salts (such as Cu sulfate) that hydrolyze completely in water, producing ionic Cu. The "insoluble" (sparingly soluble) Cu compounds act as a reservoir from which Cu ion is released to the plant surface on which it is deposited upon application.

Estimation of the world market for fungicides and bactericides by type of Cu compound and year of introduction is shown in Table 1 below (data published in 1988, Source: H. W. Richardson, Handbook of Copper Compounds and Applications*, supra).*

**Table 1 - Estimation of World Market for Fungicides/Bactericides by Type of Cu Compound**

| Cu Compound | Quantity (Tons/Yr) | % of Market | Year Introduced |
|---|---|---|---|
| Cu(II) oxychloride | 71,000 | 51.1 | 1990 |
| Cu(II) sulfate | 48,000 | 34.6 | 1761 |
| Cu(II) sulfate + lime (Bordeaux mixture) | | | 1873 |
| Cu(II) sulfate + soda ash (Burgundy mixture) | | | |
| Basic Cu(II) sulfate | | | |
| Cu(I) oxide | 6,000 | 4.3 | 1932 |
| Cu(II) hydroxide | 11,000 | 7.9 | 1960 |
| Others:Cu(II)ammonia complex., CO₃ and PO₄ | | | 1917 |

It is clear that Cu based fungicides/bactericides have been applied for a long-time and have high societal and economic impact in agriculture, water treatment and domestic living. There are approximately 2,000 registered products which contain copper compounds as active ingredients.

Several Cu compounds are registered in the United States for management of over 100 diseases on almost 50 food crops. The Cu compounds exhibit varying degrees of effectiveness for any target organism on any given host. The most common forms of Cu that satisfy these conditions to varying degrees are the normal hydrolysis products of Cu(I) and Cu(II) salts (also known as "insoluble Cu" compounds): Cu(I) oxide (Cu₂O₃), Cu(II) oxychloride (CuCl₂. 3Cu(OH)₂), tribasic Cu(II) sulfate (CuSO₄. 3Cu(OH)₂, and Cu hydroxide (Cu(OH)₂). By the mid-1930s, Bordeaux mixture was largely replaced by basic Cu(II) sulfate, Cu(I) oxide, Cu(II) oxychloride. These Cu compounds are easy to handle and relatively less phytotoxic in comparison to the Bordeaux mixture. Cu hydroxide was introduced in 1960. Kocide® 3000 is the latest Cu hydroxide based product from the DuPont Company, Wilmington, DE, which is one of the most popular fungicides/bactericides.

Currently used Cu compounds possess unique set of physical and chemical properties. They differ in their total amount of metallic Cu content and aqueous solubility. It is well understood that the antibacterial activity will depend upon the availability of soluble Cu ions in the formulation. Among the existing Cu compounds, tribasic Cu sulphates and cuprous oxide are least soluble, whereas Cu hydroxides are more soluble than Cu oxychloride.

Again, too much Cu will cause phytotoxicity and adversely affect the environment whereas sparingly soluble Cu compounds will be less effective, requiring multiple applications, thus labor extensive. A robust Cu formulation must meet at least the following three criteria: (i) the Cu release rate must be maintained at a optimum level (sustained release mechanism while minimizing phytotoxicity) to provide long-term protection against pathogen, (ii) the Cu compound must stick well to the plant surface to withstand wind blown rain so that multiple applications will not be required and (iii) the Cu compound will not cause toxicity to the environment (i.e. environmentally-safe). Due to inherent chemical and physical properties, however, the existing Cu compounds are seriously limited to meet these criteria.

With regard to copper (Cu) compounds, the efficacy of a Cu compounds can be considerably improved by reducing the particle size according to Torgeson, D.C., ed. Fungicides - An Advanced Treatise, Agricultural and Industrial Applications and Environmental Interaction. Vol. 1. (1967), Academic Press: New York, N. Y, page 697. The smaller the particle size the greater is the number of particles per gram and therefore the greater the fungicidal or bacterial activity. This welcomes nanoscience and nanotechnology that deals with matters in the nanoscale dimension, typically 1-100 nanometer range.

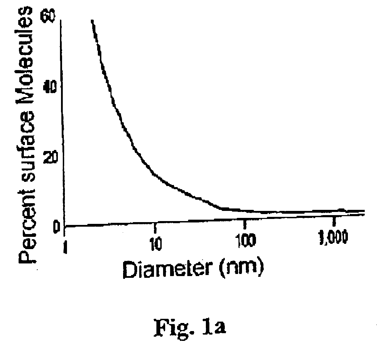

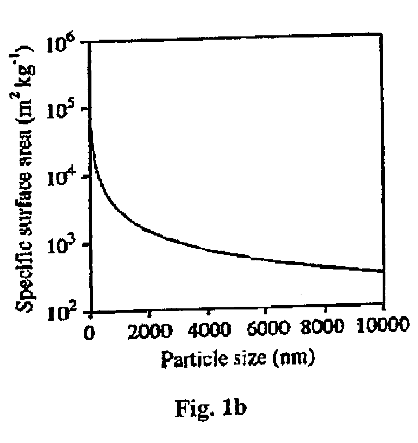

Navarro, E., et al., in "Environmental behavior and ecotoxicity of engineered nanoparticles to algae, plants, and fungi," Ecotoxicology, 2008. 17(5): pages 372-386 teaches that specific surface area as shown in Fig. 1a increases exponentially as the particle size decreases below 100 nanometers. Likewise, Oberdorster, G. et al, in Nanotoxicology: An Emerging Discipline Evolving from Studies of Ultrafine Particles, Environmental Health Perspectives, 2005. 113(7): pages 823-839 teaches that the percentage of surface atoms exponentially increase as the particle size decreases below 100 nm as shown in Fig. 1b. Thus, with smaller particles coverage is improved and there is significantly more surface area available per gram of product to release Cu ions when moisture is present.

Smaller particles also resist dislodgement better as they are lighter and have a larger surface area relative to their weight; hence a greater area of contact with the plant surface, and the result is an increase in the total force of adhesion. Cu formulations with smaller particles will therefore produce improvements in disease control through better coverage, rain-fastness, and longevity of the product and release of Cu ions on the plant surface. In this regard, some improvements in the product quality have been made on Cu compounds over the past decade. To date, however, no major breakthrough has been made that could be considered revolutionary. This strongly demands development of a new generation of Cu based fungicides/bactericides that will meet the above-mentioned criteria.

### Inhibiting Growth of Mold and Mildew

Growth of fungus, such as, mold and mildew fungi is a serious problem in warm, moist environments, such as in tropical climates. In the presence of nutrients which are found abundantly in house/building materials such as dry walls, wood, grout, carpet backing and the like, mold and mildew thrives.

Mold and mildew have similar characteristics, but are different types of microscopic fungi and are often different in color and texture and can be seen growing on objects both inside and outside buildings. Mold and mildew growth is not only detrimental to health but also presents an unsightly appearance when colonies are established on interior or exterior surfaces of buildings. *Mildew* is more often found in bathroom showers, tubs, ceramic tile grout, paper and fabric; *mold* is usually found in foods. They can be difficult to tell apart as they both use spores for reproduction. Mold is often black, green, red or blue in color, while mildew is usually gray or white. Musty and moldy odors are produced by chemical changes taking place during the mold life process and are scientifically designated as microbial Volatile Organic Compounds (mVOCs) Waste products are given off by actively growing molds.

Airborne mold spores can seriously compromise indoor air quality and cause severe allergy, asthma and other immunological problems. Health effects such as headaches, dizziness, nausea and coughing have been linked to exposure to mVOCs. Irritation of the eyes and throat may also occur as a result of breathing mold toxins. Moldy food should not be eaten. A mildew infestation on paper and some fabrics cannot be scrubbed off, but a mildew remover can usually get rid of mildew on harder surfaces, such as, surfaces in bathrooms, kitchens and on exterior walls.

Mold spores are resistant to high temperature, UV light and desiccation. The spores are abundant in the environment and remain dormant in unfavorable conditions and germinate rapidly in a favorable environment (warmth, moisture and nutrients). Bleach solutions are effective in killing both mold and mildew and are considered a very effective treatment world-wide. However, the action of bleach does not last long; multiple applications are required within one or two weeks.

U. S. Patent 3,992,146 to Fazzalari describes a process using biocidal solutions containing copper sulfate and a surfactant to inhibit growth of fungi on hard porous surfaces, such as grout.

It is desirable to use nanotechnology to expand the use of copper (Cu) loaded silica nanoparticle/nanogel formulations with superior antibacterial activity for treating areas that favor and foster the growth of mold and mildew; in addition, it is desirable for a single treatment to remain effective for from at least two to six months because of the release of ionic Cu in a slow, sustained manner and in quantities that would not violate the EPA Water Quality Standard Rule issued on December 1, 1992, setting water quality standards for copper as a priority toxic pollutant.

### Treatment of Diseases in Plants

The state of the art for methods and treatment of diseases in plants, and specifically canker in citrus plants is found in a representative sample of patents listed below:
U. S. Patent 3,983,214 to Misato et al teaches fungicidal compositions and method for protecting plants by the use of compositions which contain organic acids as an active ingredient, alkali metal salts of these organic acids, ferric citrate, ferric lactate, glycerine, aluminum chloride and esters formed between sugar and higher fatty acids having 8 to 18 carbon atoms. The compositions have no phytotoxicity and no mammalian toxicity and present no risk of pollution of soil.
U.S. Patent 5,462,738 to LeFiles et al. discloses a granular copper hydroxide dry flowable bactericide/fungicide with improved biological activity and a method of making and using.
U.S. Patent 5,939,357 to Jones et al. provides a fungicide composition which has a bicarbonate-containing inorganic salt ingredient which enhances the efficacy of a fungicide ingredient for treatment of cultivated crops.
U.S. Patent 6,471,976 to Taylor et al. discloses an improved copper complex bactericide/fungicide containing a partially neutralized polycarboxylic acid and a method of making and using.
U.S. Patent 7,163,709 to Cook et al. discloses a composition and method of providing ionic forms or compounds of any combination of three metals (copper, gold and silver) to produce a product that can be used as an antimicrobial agent, hard surface disinfectant, foliar spray or water treatment. The composition is aerosolized, misted, vaporized, fogged, humidified to produce micronized particles which are able to remain in suspension in the air for long periods of time to act on air-borne fungal spores and/or pathogens. This would be an enormously expensive composition.
U.S. Patent 7,226,610 to Winniczuk teaches compositions and methods for the treatment and prevention of disease in plants, especially citrus canker, using composition including various combinations of d-limonene, wax and monohydric alcohol.
U.S. Patent Publ. No. U.S. 200110051174 to Staats provides an antimicrobial composition containing quaternary ammonium compounds, a surfactant, a wetting agent, a 10 drying agent, a hydrophilic film forming agent, a hydrophobic water proofing agent and water, having anti viral, antibacterial, and antifungal properties, applied to plants and trees by spray coating and/or through systemic delivery to protect against harmful and destructive organisms.
U.S. Patent Publ. No. VS 2007/0087023 and U.S. Patent Publ. No. US 15 2007/0098806 both to Ismail et al disclose a polymer-based antimicrobial agent that includes a water-soluble polymer and oligodynamic metal ions (e.g., nano-sized silver particles) that interact with the water-soluble polymer to treat or prevent citrus canker.

US 2004/0096421 A1 discloses organic domain/inorganic domain complex materials comprising a composition according to the preamble of independent claim 2, and a method for synthesizing these materials according to the preamble of independent claim 1. The known hybrid materials may be used as hydrophilicity reagent and applied to the surfaces of various substances, such as a resin surface and a coated surface. The hybrid materials may also be used as a antibacterial/antifungal reagent, which may especially be used in the form of a mixture thereof with a cement.

US 2007/0140951 A1 discloses nano-sized particles, processes of making and compositions thereof. The nanoparticles are metal oxide based. One example of the metal oxide based nanoparticles are copper oxide nano crystals of the Cu/Cu₂O/CuO system. The copper oxide nanocrystals are made by a process involving thermal decomposition of copper (I) acetate at high temperature in the presence of a surfactant. This results in highly mono disperse and crystalline nano particles of Cu₂O. Evidence of Cu²⁺ in XPS and optical characterization of the nanocrystal suggested the existence of a thin layer of amorphous CuO at the nanocrystal-ligand interface.

Thus, there is a wide array of fungicidal/bactericidal compositions represented in the patent literature with formulations that include, acids, polymeric compounds, metallic ions, 20 undesirable components such as surfactants or oils. Cost, effectiveness and bacterial or fungal resistance to treatment are also considerations.

With regard to citrus canker, a particularly acute and critical situation has developed in the United States. Citrus canker is a bacterial disease of citrus that causes premature leaf and fruit drop. Symptoms of the disease are exhibited on leaves and fruit by brown, raised lesions surrounded by an oily, water-soaked margin and a yellow ring or halo. Old lesions in leaves may fall out, creating a shot-hole effect.

Citrus canker does not harm humans or animals or plant life other than citrus. However, citrus canker affects all types of citrus, including oranges, sour oranges, grapefruit, tangerines, lemons and limes. Canker causes the citrus tree to continually decline in health and fruit production. Ultimately, the tree will produce no fruit at all.

Citrus canker is highly contagious and can be spread rapidly by windborne rain, lawnmowers and other landscaping equipment, people carrying the infection on their hands, clothing, or equipment, moving infected or exposed plants or plant parts.

The United States Department of Agriculture (USDA) withdrew eradication program funding due to the impacts of legal constraints and the 2004/2005 hurricanes that caused canker to spread so far that eradication was no longer possible.

Florida is currently under a statewide quarantine by the USDA and no citrus may leave the state unless the USDA has issued a limited permit. No Florida grown citrus may enter any citrus producing states or territories. No citrus plants or parts may enter or exit Florida.

Over 16 million trees have been destroyed in Florida since canker was first identified in Florida in 1910. Twice the state of Florida has declared that canker was eradicated, in 1933 and again in 1994, only to have the canker disease resurface a third-time in 1995 near Miami International Airport. In 2008, the citrus industry in Florida is now quarantined because of canker disease that was spread by the 2004/2005 hurricanes. Once productive groves, land and trees are becoming unproductive at an enormous cost to the state and its citizens. The number of commercial groves in Florida dropped from about 857,000 acres in 1996 to 621,000 acres in 2006, according to H. Florin in "The Sour State of Florida Citrus," published on line at http://www.time.com/time/nation/article/0,8599,1836766,00.html for Time Magazine, August 28, 2008.

Worldwide, citrus canker is one of the most devastating diseases that has seriously affected the citrus industry in over thirty countries in Asia, the Pacific and Indian Ocean islands, South America, and the Southeastern USA, as reported by A. K. Das in "Citrus Canker-A review," J. Appl. Hort. 2003, 5 (1), 52-60. This disease is caused by the bacterium *Xanthomonas axonopodis* pv. *Citri*, a gram-negative bacterium. The pathogen causes necrotic lesions on leaves, stems and fruit. Severe infections can cause defoliation, badly blemished fruit, premature fruit drop, twig dieback and general tree decline, as discussed by T. S. Schubert et al. in Plant Disease, 2001, 85 (4). The tenaciousness of citrus canker disease and infection is discussed by C.H. Bock, et al. "Efficacy of Cankerguard(R) Sprays for Effective Decontamination of Citrus Canker." in Phytophology 2009, 99, (6), page S13-Abstract of Presentations, 2009 APS Annual Meeting. Bock et al. found that after treatment, infection took place, at or very soon after, inoculation as decontamination did not reduce disease incidence or severity.

The most serious consequence of citrus canker infestation is the impact on commerce resulting from restrictions to interstate and international transport and sale of fruit originating from infested areas, causing huge economic loss every year. Citrus growers are aggressively looking for a robust remedy.

There remains a continuing and urgent need for the development of new and more effective fungicides/bactericides that are inexpensive and easy to manufacture which possess preventive, curative and systemic activity for the protection of not only citrus trees, but all cultivated plants, with a minimum of phytotoxic side effects. The present invention uses nanotechnology to meet the need for an inexpensive, more effective fungicide/bactericide.

Further innovative developments of the present invention are based on Cu loaded silica nano gel (CuSiNG). In comparison to commercially available products, the nanogel technology herein has demonstrated its superiority in improving Cu bioavailability, plant surface coverage and longevity and provides a means for detecting when the CuSiNG needs to be reapplied for maximum protection.

### SUMMARY OF THE INVENTION

The present invention provides a method for synthesizing a silica-based nanoformulation according to independent claim 1, and a composition for treating and preventing disease in a plant species according to present claim 2. Dependent claims 3 to 5 are directed to preferred embodiments of the composition of the present invention.

A first objective of the present invention is to provide a composition and method for fabricating a silica-based nanoformulation that provides a unique nanoenvironment to host antibacterial and antifungal agents.

A second objective of the present invention is to provide a silica-based nanoformulation that provides a unique nanoenvironment to host antibacterial and antifungal agents with controlled release kinetics, establishing a sustained release mechanism.

A third objective of the present invention is to provide a silica-based nanoformulation that pro vi des a unique nanoenvironment to host antibacterial and antifungal agents (Anti-B/F) to enhance the efficacy of the Anti-B/F agents.

A fourth objective of the present invention is to provide a nanoformulation consisting of silica nanoparticle (SiNP), or silica nanogel (SiNG) or a combination of SiNP and SiNG.

A fifth objective of the present invention is to provide a simple, cost-effective fabrication or synthesis method for a silica-based nanoformulation with antibacterial and antifungal content that can be prepared on-site, in a few hours, in a single-pot synthesis method, requiring no purification steps.

A sixth objective of the present invention is to provide a simple, cost-effective fabrication or synthesis method for a concentrated silica-based nanoformulation with antibacterial and antifungal content that can be prepared on-site, diluted for field application by a non-technical person.

A seventh objective of the present invention is to provide a silica-based nanoformulation with antibacterial and antifungal content for treating a citrus canker disease-prone surface by providing uniform coverage to that surface, surface adherence for a satisfactory period of time, sustained release over time, better control of Anti-B/F release, a reduction in toxicity and stress to the environment in the treated area.

An eighth objective of the present invention is to provide a silica-based nanoformulation with antibacterial and antifungal content for treating a citrus canker disease-prone surface with a broad array of benefits, including, but not limited to, surface adherence for a satisfactory period of time, sustained release over time, control of Anti-B/F release, reduced toxicity and stress to the environment in the treated area.

A ninth objective of the present invention is to provide a silica-based nanoformulation with antibacterial and antifungal content for treating citrus canker and preventing canker disease for a period of from five to nine months.

A tenth objective of the present invention is to provide a silica-based nanoformulation with antibacterial and antifungal content for the agriculture industry (vegetables, flowers, grass and other plants), household applications (fabrics, leather, plastics, paint and the like).

An eleventh objective of the present invention is to provide a copper (Cu) loaded, silica-based nanoformulation with residual fertilizing properties for soil wherein leaves and branches of plants treated with the nanoformulation continue to release Cu nutrients when the leaves and branches drop to the ground.

A twelfth objective of the present invention is to provide a copper (Cu) loaded, silica-based nanoformulation with residual fertilizing properties for soil wherein when plants and trees treated with the nanoformulation herein are harvested for mulch or compost, the soil is enriched with residual Cu ions.

Another objective of the present invention is to provide a copper (Cu) loaded, silica-based nanoformulation with superior antifungal properties to inhibit the growth of mold and mildew for at least six months by the release of a fungicide in a slow and sustained manner.

In addition to the objectives stated above, innovative developments and improvements using Cu loaded silica nanogel (CuSiNG) include a further objective of reducing bacterial populations on plant and foilage surfaces by improving copper bioavailability.

Another objective of the present invention is to optimize copper (Cu) loading of multiple valence forms (Cu⁺¹ and Cu²⁺ states) as well as its crystalline (Cu oxide) and amorphous (complexed with silica matrix) forms of copper within the silica nanogel to control copper release kinetics.

It is also an objective of the present invention to provide copper in a CuSiNG spray application with plant residence longevity wherein the CuSiNG diffuses out and covers newly exposed tissue surface of rapidly growing young fruits and leaves, thus minimizing the number of applications per growing season.

Another objective of the present invention is to provide a fluorescent silica nanogel, incorporating a ruthenium based dye (RuCuSiNG) to provide a means for measuring the diffusion property of CuSiNG, visually observing the adherence property of CuSiNG and determining when reapplication of CuSiNG is warranted.

A preferred method for synthesizing a silica-based nanoformulation containing antibacterial and antifungal agents using a one-pot, acid-catalyzed, sol-gel process, includes selecting a reaction vessel containing an acidic reaction medium, adding tetraethoxysilane (TEOS), water, ethanol and an antibacterial or antifungal agent containing copper ions to the reaction vessel with the acidic medium to form mixture I, allowing the reaction of mixture I to continue for approximately one hour to facilitate the formation of a plurality of silica-based nanoparticles, adding a neutralizing agent after the formation of a plurality of nanoparticles to adjust the pH value of the reaction mixture to approximately 7.0, allowing the neutralized reaction of mixture I to continue for approximately 12 hours, preferably overnight, to allow gelation to occur, wherein the nanogel structure consists of interconnected nanoparticles; and collecting a porous silica-based nanogel having antibacterial and antifungal agents bound in a plurality of interconnected nanoparticles of the nanogel.

The preferred acidic reaction medium of the method is an aqueous solution of an acid selected from one of hydrochloric acid, sulfuric acid and nitric acid. The preferred antibacterial or antifungal agent is a plurality of copper (Cu) ions sourced from a copper compound.

It is also preferred that the reaction of mixture I occur at ambient temperatures and pressures.

It is preferred that the plurality of interconnected nanoparticles of the nanogel contain a plurality of Cu ions electrostatically bound to the nanoparticle core, a plurality of Cu ions covalently bound to a hydrated shell surface and nanopores of the nanoparticle, and a plurality of Cu oxide/hydroxide as nanoclusters/nanoparticles bound to the surface of the nanoparticle.

A preferred composition and method for treating and preventing disease in a plant species consists of a silica-based nanoformulation, preferably containing a plurality of copper ions embedded in a plurality of silica nanoparticles and more preferably, a plurality of copper ions embedded in a silica nanogel. Preferably the plurality of copper ions is from copper compounds, such as, metallic copper, copper oxide, copper oxychloride, copper sulfate, copper hydroxide and mixtures thereof.

Preferably the diseased plant treated by the preferred composition is a member of the citrus species, wherein the disease is citrus canker caused by any species of Xanthomonas.

It is preferred that during treatment, the plurality of copper (Cu) ions is released in a sustained manner for long-term treatment of the plant for a time period of at least six months.

A preferred method for treating or preventing disease in a plant species comprising applying to the plant species a composition that comprises a silica-based nanoformulation, wherein the preferred composition further comprises a plurality of copper ions embedded in a plurality of silica nanoparticles, more preferably, a plurality of copper ions embedded in a silica nanogel.

The composition further includes a plurality of copper ions from copper compounds, such as, metallic copper, copper oxide, copper oxychloride, copper sulfate, copper hydroxide and mixtures thereof.

A preferred method for treating and inhibiting the growth of mold and mildew on a surface selected from the group consisting of interior and exterior walls of buildings, ceilings, ceramic tile grout, and bathroom fixtures comprising applying to the surface, a composition that comprises a silica-based nanoformulation. The preferred silica-based nanoformulation is a plurality of interconnected nanoparticles of the nanogel contain a plurality of Cu ions electrostatically bound to the nanoparticle core, a plurality of Cu ions covalently bound to a hydrated shell surface and nanopores of the nanoparticle, and a plurality of Cu oxide/hydroxide as nanoclusters/nanoparticles bound to the surface of the nanoparticle.

The preferred source of the plurality of Cu ions is selected from metallic copper, copper oxide, copper oxychloride, copper sulfate, copper hydroxide and mixtures thereof.

A preferred method for adding nutrients to the soil includes treating vegetation with a copper (Cu) loaded silica nanoparticle formulation, allowing leaves and branches to drop to the ground, and leaving the treated leaves and branches to biodegrade and thereby fertilizing the soil with Cu ion nutrients in a slow-release, non phytotoxic manner.

Another preferred method for adding nutrients to soil includes treating vegetation with a copper (Cu) loaded silica nanoparticle formulation, harvesting and comminuting the vegetation to form mulch or compost, and spreading the mulch or compost over the soil to allow the leaching of Cu ion nutrients in a slow-release, non-phytotoxic manner with exposure to atmospheric conditions selected from at least one of rain, wind, snow, and sunlight.

A preferred silica based nanoformulation is provided wherein a silica matrix has a copper ion loaded silica nanogel (CuSiNG) that simultaneously hosts two active forms of copper wherein a first form of copper is amorphous and a second form of copper is crystalline and the equilibrium established by the two active forms of copper can be adjusted to control copper ion release kinetics and improve copper ion bioavailability.

Another preferred silica based nanoformulation exhibits a property wherein a plurality of copper ions diffuse out from a location of application on a plant tissue surface in a moist environment and cover the exposed tissue surface of rapidly growing fruit and leaves, thereby minimizing the application frequency per growing season and increasing plant surface coverage and longevity of coverage.

It is also preferred that the silica based formulation of the present invention further include a silane precursor compound to achieve uniform plant surface coverage, modulate copper ion release rate, improve rain-fastness and increase longevity of plant surface coverage. A preferred silane precursor compound is 3-(trihydroxysilyl) propyl methylphosphonate (THPMP).

Another preferred silica based nanoformulation includes a ruthenium based fluorescent dye (RuCuSiNG) to assess adherence properties of silica nanogel (SiNG) at microscopic levels and a preferred ruthenium based fluorescent dye is tris(2,2'-bipyridyl dichlororuthenium) hexahydrate (Rubpy) and the ruthenium based fluorescent dye, tris(2,2'-bipyridyl dichlororuthenium) hexahydrate (Rubpy), is used to monitor copper ion release from copper loaded silica nanogel (CuSiNG) in real time.

In addition the preferred ruthenium based fluorescent dye, tris(2,2'-bipyridyl dichlororuthenium) hexahydrate (Rubpy), is used to measure the amount of copper loaded silica nanogel (CuSiNG) remaining on the surface of a plant to determine if a repeated application is required.

A more preferred hybrid silica based nanoformulation is provided wherein a silica matrix includes a plurality of copper ions and a silane precursor, 3-(trihydroxysilyl) propyl methylphosphonate (THPMP), loaded into the copper loaded silica nanogel (CuSiNG) to achieve uniform plant surface coverage and provide a surface coverage that expands with plant growth.

Further objects and advantages of this invention will be apparent from the following detailed description of a presently preferred embodiment that is illustrated schematically in the accompanying drawings.

### BRIEF DESCRIPTION OF THE FIGURES

Fig. 1a is a graph showing the exponential increase of surface molecules as a function of decreasing particle size. (Prior Art)
Fig. 1b is a graph of the relationship between specific surface area of a spherical particle and the size of the particle (diameter in nm). (Prior Art)
Fig. 2 is a schematic representation of the silica-based nanoformulation of the present invention showing a copper (Cu) loaded silica nanoparticle (CuSiNP).
Fig. 3 is a transmission electron microscopy (TEM) image of copper loaded silica nanogel (CuSiNG) formulation consisting of silica nanoparticles approximately 10 nm in diameter that are interconnected.
Fig. 4a shows the copper (Cu) loaded silica nanogel (SiNG) material dispersed in water.
Fig. 4b shows the water dispersible CuSiNG after centrifugation with the separation of residue from the supernatant liquid.
Fig. 4c shows a water-dispersible CuSiNG mixture treated with ethylenediamine tetraacetic acid (EDTA, a strong chelating agent) forming a water-soluble Cu-EDTA complex.
Fig. 4d shows the water-soluble Cu-EDTA complex after centrifugation with the separation of the Cu-EDTA complex and a residue of silica nanogel (SiNG).
Fig. 5 is an X-ray Diffraction (XRD) pattern of a CuSiNG sample and a commercially available bactericide/fungicide, DuPont™ Kocide® 3000.
Fig. 6a is the high resolution X-ray photoelectron spectroscopy (XPS) spectrum of CuSiNG sample showing Cu 2p peaks.
Fig. 6b is the X-ray photoelectron spectroscopy (XPS) of a CuSiNG sample showingCu 2p core level spectrum without satellite peaks.
Fig. 6c is the X-ray photoelectron spectroscopy (XPS) spectrum for oxygen (O) 1s in a CuSiNG sample.
Fig. 6d is the X-ray photoelectron spectroscopy (XPS) spectrum for silicon (Si) 2p³ in a CuSiNG sample.
Fig. 7a is a drawing of a disc diffusion assay of CuSiNG dispersed in de-ionized water having a pH of approximately 7.
Fig. 7b is a drawing of a disc diffusion assay of copper sulfate (CuSO₄) solution in deionized water.
Fig. 7c is a drawing of a disc diffusion assay of DuPont™ Kocide® 3000 fungicide/bactericide dispersed in de-ionized water.
Fig. 8 is a digital image of a lemon tree leaf taken 5 days after the initial application of a silica-based nanoformulation of the present invention (CuSiNG) is exposed to heavy rain, wind and thunderstorm conditions in an outdoor environment.
Fig. 9a shows digital field image of CuSiNG on the leaf surface *before* rain water is sprayed on the leaf surface.
Fig. 9b is a drawing of the fluorescent image of the leaf in Fig. 9a showing adherence of CuSiNG before rain water is sprayed on the leaf surface.
Fig. 9c shows a digital field image of CuSiNG on the leaf surface *after* approximately 5 minutes of continuous rain water spray onto the leaf surface.
Fig. 9d is a drawing of the fluorescent image of the leaf in Fig. 9c showing adherence of CuSiNG *after* approximately 5 minutes of continuous rain water spray onto the leaf surface.
Fig. 10a shows the formation of an interconnected network like structure of CuSiNG.
Fig. 10b shows the appearance of scattered dark areas in the silica matrix, confirming the presence of electron-rich materials in the amorphous matrix.
Fig. 10c shows the selected area electron diffraction (SAED) pattern of CuSiNG which indicates the formation of amorphous CuSiNG.
Fig. 10d is a high resolution transmission electron microscopy (HRTEM) image revealing the formation of 1-3 nanometer (nm) dark contrast particles of Cu₂O crystallites, confirmed by lattice spacing measurement, embedded in an amorphous silica matrix.
Fig. 11a is an image of CuSiNG treated disc (disc diffusion assay) showing an area of no *Xanthomonas alfalfa (X. alfalfae), a citrus canker surrogate* growth surrounding the central disc.
Fig. 11b is an image of Kocide 3000® treated disc (disc diffusion assay) disc showing an area of no *X. alfalfae* growth surrounding the central disc saturated with Kocide 3000®, wherein the area of no *X. alfalfae* growth surrounding the central disc is less than the area of no growth surrounding the disc treated with CuSiNG of identical metallic Cu content in Fig. 11a.
Fig. 11c is an image of Cu-sulfate treated disc (disc diffusion assay) showing an area of no *X alfalfae* growth surrounding the central disc saturated with Cu-sulfate, wherein the area of no *X. alfalfae* growth surrounding the central disc is less than the area of no growth surrounding the disc treated with CuSiNG of identical metallic Cu content in Fig. 11a and the disc treated with Kocide 3000® of identical metallic Cu content in Fig. 11b.
Fig. 11d is an image of silica nanogel (SiNG) treated disc (disc diffusion assay) disc showing growth of *X*. *alfalfae*completely surrounding the central disc treated with SiNG, thus confirming that SiNG has no antibacterial properties.
Fig. 12a is a fluorescence image of a blank disc on a nutrient agar plate.
Fig. 12b is a fluorescent image of a RuSiNG treated disc on the nutrient agar plate of Fig. 12a after refrigeration for 7 days.
Fig. 12c is a fluorescent image of the nutrient agar plate of Fig. 12b with the RuSiNG treated disc removed.
Fig. 13a is a drawing of a fluorescent dye (Rubpy) loaded SiNG (RuSiNG) material applied to a leaf surface prior to washing the leaf.
Fig. 13b is a drawing of a leaf surface to which RuSiNG is applied after a one minute (1 min) wash under a fast-running faucet.
Fig. 13c is a drawing of a leaf surface with an application of RuSiNG after a 6 minute wash under a fast-running faucet.
Fig. 13d is a drawing of a leaf surface with an application of RuSiNG after a 16 minute wash under a fast-running faucet.
Fig. 14a shows a centrifuged tube of RuCuSiNG on the left and on the right a test tube of EDTA treated centrifuged RuCuSiNG material as it appears under ambient light.
Fig. 14b shows a centrifuged tube of RuCuSiNG on the left and on the right a test tube of EDTA treated centrifuged RuCuSiNG material as it appears under illumination from a hand-held 366 nm UV excitation source.

### DESCRIPTION OF THE PREFERRED EMBODIMENT

Before explaining the disclosed embodiment of the present invention in detail it is to be understood that the invention is not limited in its application to the details of the particular arrangement shown since the invention is capable of other embodiments. Also, the terminology used herein is for the purpose of description and not of limitation.

It would be useful to discuss the meanings of some words and abbreviations used herein to explain the invention in greater detail.

Cu stands for the metallic element copper.

CuSiNG stands for Copper loaded Silica NanoGel

CuSiNP stands for Copper loaded Silica NanoParticle

Kocide® 3000 fungicide/bactericide; Kocide is a registered **trademark** of E.I. du Pont de Nemours and Company.

NG stands for Nanogel, which is the gel-like substance formed by the interconnection of nanoparticles.

NP stands for Nanoparticles which have a particle size from approximately 10 nm to approximately 50 nm

Ru stands for Rubpy, a ruthenium-based fluorescent dye, tris(2,2'-bipyridyl dichlororuthenium) hexahydrate [Ru(bpy)]. Any fluorescent dye or fluorescent nanoparticle (such as quantum dots) that undergoes fluorescence quenching in presence of Cu or any other such antibacterial/antifungal active agent/ingredient is suitable for use herein.

Si is used herein to mean silicon dioxide, which is also commonly known as "silica."

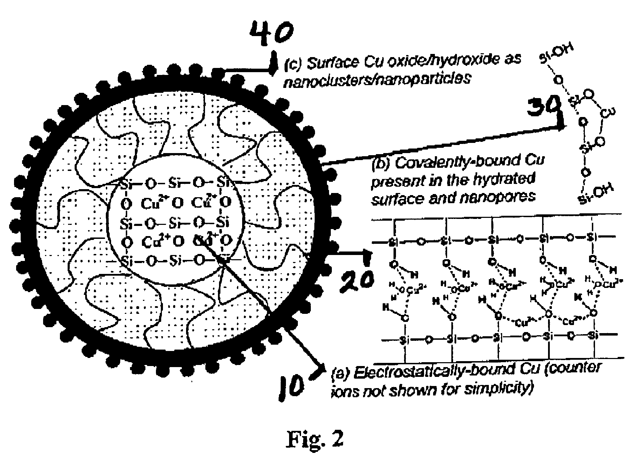

The present invention involves a unique application of nanoscience and nanotechnology to develop engineered copper Cu(II) loaded silica nanogel (CuSiNG) material. The nanotechnology allows the manipulation of a silica nanoenvironment around Cu, establishing a sustained Cu ion release mechanism. The novel CuSiNP design shown in Fig. 2, allows association of copper (Cu) to silica NG in three different forms, enabling sustained Cu release. First, electrostatically bound Cu ions in the core **10.** Second, covalently bound Cu in the hydrated particle surface **20** and nanopores **30.** Third, surface bound Cu as hydroxide/oxide nanoclusters/nanoparticles **40.** The nanogel structure consists of ultra-small nanoparticles (CuSiNPs) less than approximately 50 nm in diameter to provide better plant surface coverage and improved adherence properties. All these attractive features will form a solid basis for long-term protection of plants from pathogens using CuSiNG material.

Due to nanoscale engineering, the CuSiNG of the present invention has the following advantages over the existing Cu based compounds: uniform coverage of plant surface because of ultra-small particle size, better adherence property due to gel-like nanostructure, sustained (long-term) Cu release profile, better control on Cu release rate (adjustable "soluble" to "insoluble" Cu ratio), more antibacterial/antifungal activity with less amount of Cu content, reduced phytotoxic effect because of adjustable "soluble" to "insoluble" Cu ratio and environment-safe due to less Cu content, no harmful by-product formation, water-based synthesis, utilization of excess CuSiNG as plant nutrient and minimal possibility of having elevated local Cu concentration that could cause environmental toxicity.

The synthesis protocol has the following advantages: (i) simplicity, (ii) water-based, (ii) scalable to field applications, (iii) single-pot synthesis method, requiring no purification steps and (v) concentrated CuSiNG material could be easily diluted for field application. A non-technical person can do this task by adding an appropriate amount of water, thus reducing shipping costs. The method also uses inexpensive raw chemicals and is easily produced in a cost-effective manner.

### Example 1 - Synthesis of CuSiNG

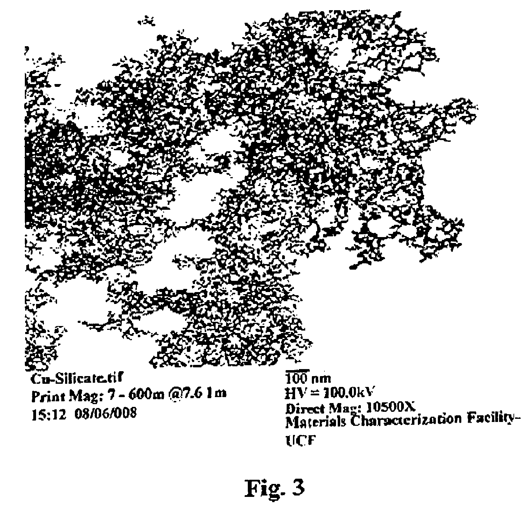

The synthesis of the copper/silica nanogel (CuSiNG) was carried out at room temperature via one-step acid-catalyzed sol-gel process using tetraethoxysilane (TEOS), water, ethanol, and Cu(II) sulfate. Hydrolysis as shown below in equation 4 and condensation reactions in equations 5 and 6 below resulted in the formation of ultra-small (<10 nm size) Cu loaded silica nanopaticles (CuSiNPs). When maintained in acidic condition for several hours, gelation takes place. Fig. 3 shows formation of CuSiNG where CuSiNPs are interconnected.

**(RO)₃SiOR + H₂O --> (RO)₃SiOH + ROH** **(4)**

**2(RO)₃SiOH --> (RO)₃Si-O-Si(OR)₃ + H₂O** **(5)**

**(RO)₃SiOH + ROSi(OR)₃ --> (RO)₃Si-O-Si(OR)₃ + ROH** **(6)**

The overall synthesis process will involve two simple steps: First, the addition of Cu(II) salts to the acidic reaction medium in the beginning of nanoparticle synthesis and second, addition of a neutralizing agent, such as, sodium hydroxide (NaOH) after the synthesis to adjust the pH to 7.0. The acid reaction medium is formed with any inorganic acid, such as hydrochloric (HCl), sulfuric (H₂SO₄), nitric (HNO₃) and the like. The neutralizing agent is any compound with an alkaline pH, such as sodium hydroxide, ammonium hydroxide, potassium hydroxide and the like.

Gelation of sol particles will take place over time. The viscosity of the resulting gel can be easily controlled by adjusting the pH and timing between these two simple synthesis steps. This is a simple one-pot, a few hours long synthesis technique that requires no purification. Because of this simplicity, large scale, multi-ton scale production of CuSiNG is feasible. The process is environmentally-safe because it is a water-based "Green synthesis" technique, resulting in no harmful by-products, both silica and Cu are naturally found in the environment, CuSiNG will have "insoluble" Cu at low levels, and CuSiNG will not elevate local Cu levels in the soil as it will be consumed as a plant nutrient. The method also uses readily available inexpensive chemicals for the synthesis of CuSiNG.

To study the binding of ionic Cu to SiNG, CuSiNG is synthesized in acidic pH reaction media, such as a 1 % hydrochloric acid solution; however, a neutralizing agent is not added. The CuSiNG material is collected by centrifugation to remove the supernatant containing any free Cu ions. The CuSiNG is washed several times with deionized (DI) water. In this CuSiNG material, CuSiNP surface will be free of Cu hydroxide/oxide precipitate and Cu will be found only in its ionic form and is hereafter identified as "CuSiNP-clean."

The CuSiNG (nanogel) consists of inter-connected ultra-small uniform size CuSiNPs (particles), as shown in Fig. 2, forming a gel-like structure, as shown in Fig. 3. These CuSiNPs will have core-shell nanostructure. The core **10** will consist of Cu ion loaded silica and the shell **20** will support Cu hydroxide/Cu oxide nanoclusters **40** that will be deposited on the surface of the shell **20.** The CuSiNP size is tunable and the particle size is variable from 10 nm to 50 nm by controlling nucleation and growth process. The rationale of varying particle size is to control the surface to volume ratio. Larger size particle will have less surface area but more core volume. This will provide a unique opportunity to manipulate the ratio of ionic Cu in the CuSiNP core **20** to the surface-bound ionic Cu **40.** It should also be noted that amorphous silica is highly porous, consisting of numerous hydrated nano-channels **30.** The channel diameter is typically 1-2 nm. Water and ethanol molecules populate the plurality of nano-channels (bound via hydrogen bonding) and provide a unique nanoenvironment to Cu ions. It should be noted that the polarity and the hydrogen bonding capacity can be easily tuned within the nano-channel **30** by varying ethanol to water ratio, providing a good control over Cu ion release from the channels. The core **10** of the particle has a reduced polar environment and is deprived of solvent molecules. This environment will facilitate the formation of Cu salt nanocrystals where both Cu and sulfate ions will be present in a reduced polar environment, as shown in Fig. 2.

In CuSiNG, surface-bound ionic Cu will populate the particle surface as well as at the particle-particle interface. Therefore, the unique CuSiNP design provides three different Cu nanoenvironments, (i) electrostatically held core **10** Cu; i.e. ionic Cu at the NG core, (ii) covalently bound ionic Cu within nano-channels **30,** at the particle surface **20** and at the interface and (iii) deposited Cu on the particle surface in its hydroxide/oxide form **40;** the surface form **40** of Cu will be highly exposed to the surrounding environment. The Cu present in these three nanoenvironments will establish different Cu release kinetics that can be controlled in a sustained manner. The CuSiNG is expected to have very uniform coverage on plant surface due to smaller particle size. Again, much improved surface adherence property is expected because of gel-like nanostructure of CuSiNG (as it takes the advantage of combined adherence property of individual CuSiNPs in the network).

The novel CuSiNG design provides more soluble ("free and active") Cu for improved antifungal/antibacterial activity even though it will have less metallic Cu content per gram of material. During spray application in the field, some amount of CuSiNG will be deposited on the soil that will serve as Cu-based nutrient for the plant. Reduced Cu content in CuSiNG will thus be used for dual purposes; first as an essential micronutrient and secondly, as a fungicide/bactericide while minimizing Cu related toxicity in the environment.

### Example 2 - Binding of Copper to Silica Nanogel

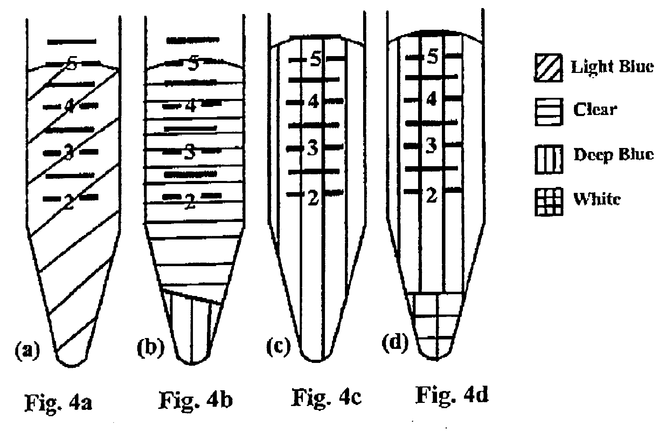

The binding of Cu to SiNG was clearly evident from the blue color appearance of the product shown in Fig. 4a. The product is water-dispersible. Upon centrifugation, a blue residue was obtained and the supernatant was clear as shown in Fig. 4b. In contrast, silica NG when prepared in the absence of Cu ions showed characteristic white color of silica.

To evaluate how well Cu was bound to silica NG, CuSiNG was treated with ethylenediamine tetraacetic acid (EDTA), a strong chelator for Cu ions. Within several minutes, a water-soluble Cu-EDTA complex formed as shown in Fig. 4c. Upon centrifugation, we obtained white color residue of silica NG and blue color supernatant of Cu-EDTA complex as shown in Fig. 4d. These results clearly demonstrate that the binding of Cu to silica is not as strong as in Cu-EDTA complex. Therefore, the loaded Cu could be slowly released in its ionic form from the CuSiNG.

### CuSiNG Product Characterization.

Samples were characterized by transmission electron microscopy (TEM), X-ray diffraction (XRD), X-ray photoelectron spectroscopy (XPS), and energy dispersive spectroscopy (EDS).

### Size

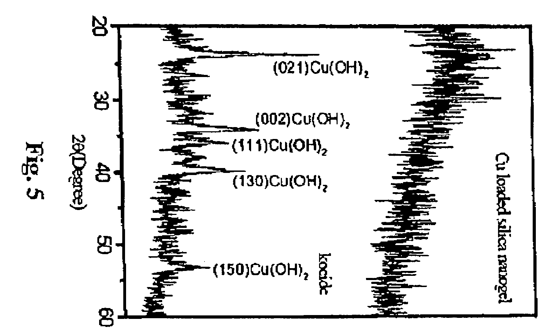

The size of CuSiNG was determined using transmission electron microscopy (TEM). The CuSiNG consisted of ultra-small (∼10 nm) size inter-connected nanoparticles (NPs), forming a gel-like nanostructure as shown in Fig. 3. X-ray diffraction (XRD) was used to determine crystallinity (if any) for CuSiNG and compared the data with Kocide® 3000. XRD pattern clearly distinguishes CuSiNG from Kocide® 3000. As shown in the Fig. 5, Kocide® 3000 was primarily crystalline Cu hydroxide/oxide material whereas CuSiNG was purely amorphous in nature. The scanning electron microscopy-energy dispersive spectroscopy (SEM-EDS, an elemental analysis technique) showed that the amount of Cu (in atomic percentage, qualitative estimation) present in Kocide® 3000 and CuSiNG is 54 at.% and 13 at.%, respectively. The oxidation state of Cu in CuSiNG was determined by the X-ray photoelectron spectroscopy (XPS). XPS is able to provide information on the oxidation state of each component in the sample as well as the composition of the sample surface.

### Binding energy (eV)

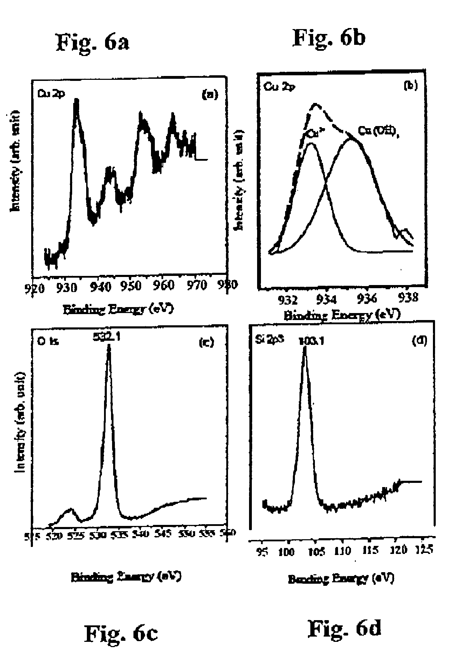

High resolution regional spectra of the key elemental composition provide detailed individual component information such as chemical shift and intensity changes, indicating changes of bonding among individual elements. High-resolution XPS spectra of copper (Cu) 2p, oxygen (O) 1s and siica (Si) 2p3 are shown in Fig. 6. The Cu 2p peaks at binding energy 933.3 eV was attributed to the Cu²⁺ environment. Broad satellite peaks at higher binding energies also suggest the presence of Cu²⁺ ionic states. The higher binding energy component located at 935.2 eV was attributed to Cu(OH)₂ species. The O 1s and Si 2p3 peaks were located at binding energy of 532.1 eV and 103.1 eV respectively. The binding energy of Si 2p matches well with the data published by National Institute of Standard and Technology of USA that is the Si in the CuSiNG sample is in the form of SiO₂.

### Compositional Analysis

Semi-quantitative compositional analysis is possible by comparing XPS and EDS data, since XPS is a surface sensitive method and EDS is a bulk method of compositional analysis. The atomic ratios of Si/Cu from XPS and EDS were determined by integrating the peak areas and dividing by the sensitivity factors. The Si/Cu ratios from EDS and XPS data were identified as 2.3 and 7.3, respectively, confirming that Cu is distributed throughout the CuSiNG.

### Antibacterial Activity

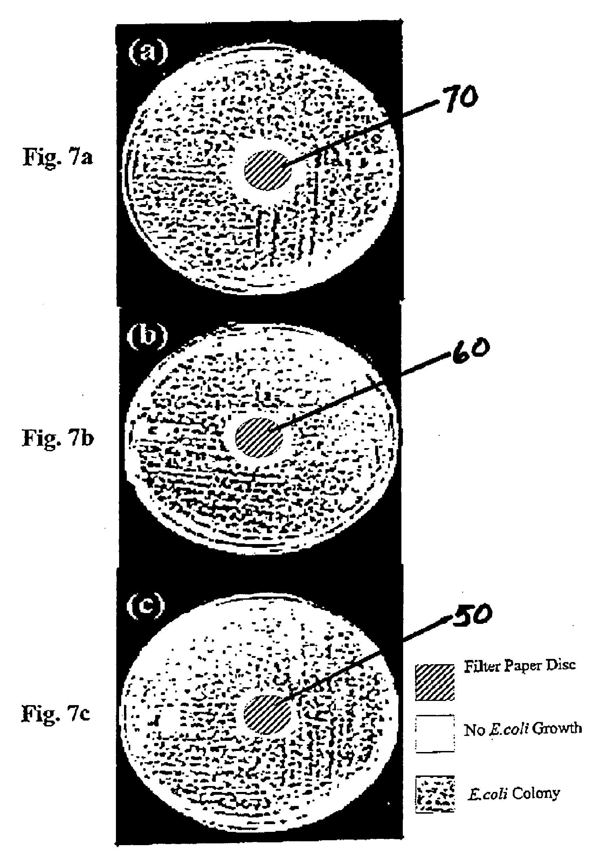

Assessment of antibacterial activity of CuSiNG was performed using *E-coli* as a model system. Disc diffusion assay, considered as one of the most common microbiological methods for the evaluation of potential antibiotic agents, was used to evaluate antimicrobial activity of CuSiNG material. Specifically, *E. coli* (strain 8739 from ATCC) grown on nutrient agar plates were incubated with filter paper discs containing equal volume and Cu concentration of aqueous solutions of either Kocide® 3000 **50,** Cu sulfate (pH 7) **60,** CuSiNG (pH 7) **70** or pure silica nanoparticles (NPs) (pH 7) (not shown). After an overnight incubation at 37°C, we found that the CuSiNG exhibited very significant antimicrobial activity, as shown in Fig. 7a, and indicated by a marked zone of clearance extending away from the corresponding disc **70.** Notably, the activity of the CuSiNG was more pronounced than that of the Cu sulfate solution on disc **60,** as shown in Fig. 7b and Kocide® 3000 on disc **50,** shown in Fig. 7c even though the Cu content in CuSiNG is about 4 times less than the Cu content in Kocide® 3000 (as determined by the SEM-EDS measurements). The area of no *E. coli* growth surrounding the filter paper disc **50** with Kocide® 3000 is approximately half the area of no *E. coli* growth for the disc **70** with CuSiNG, which confirms the efficiency of the CuSiNG formulation with one-fourth of the copper content, the CuSiNG formulation has twice the antimicrobial activity as Kocide® 3000. It should be noted that pure silica NG, prepared without the Cu ions, did not show any antibacterial activity.

### Adherence Property

Assessment of the adherence property of CuSiNG was qualitatively evaluated on a lemon plant. The plant was obtained from a Home Depot® hardware store in Orlando, FL. In a typical experimental procedure, a first spraying of an aqueous suspension of CuSiNG and Kocide® 3000 was uniformly sprayed at a distance of 6 inches over half a dozen preselected experimental leaves, assigned for each sample, using a hand-held spray bottle. The excess liquid dripped off the leaf surface. At this stage, digital images were taken of the leaf surface sprayed with Kocide® 3000, CuSiNP and CuSiNG, the adherence results are recorded in Table II below. The sprayed liquid was allowed to completely dry and again images were taken of the dried leaf surface sprayed with Kocide® 3000 and CuSiNG. About 2 hours later, natural rain water was sprayed continuously for approximately 5 minutes onto the same experimental leaves and the leaves with a continuous spraying of rain water were allowed to dry completely. At this stage, digital images of the dry leaves having a first spraying of Kocide® 3000, CuSiNP and CuSiNG and a second spraying of rain water are evaluated visually for adherence of antibacterial/antifungal agent to the surface of the leaf. A scale of 1 to 5 is used to evaluate adherence.
1 = 1-5% antibacterial/antifungal composition is on the surface of the leaf;
2 = 6-15% of antibacterial/antifungal composition is on the surface of the leaf;
3 = 16- 40% of antibacterial/antifungal composition is on the surface of the leaf;
4 = 41 - 75% of antibacterial/antifungal composition is on the surface of the leaf;
5 = 76 - 95% of antibacterial/antifungal composition is on the surface of the leaf.

**Table II - Adherence of Antibacterial/Antifungal Composition to Leaf Surface**

| PRODUCT | KOCIDE® 3000 | CuSiNP | CuSiNG |
|---|---|---|---|
| First spraying (Product applied) | 5 | 5 | 5 |
| Drying | 4 | 4 | 5 |
| Second spraying (5 minutes rainwater) | - | - | - |
| Drying | 2 | 3 | 4 |
| Product on Leaf | 1 | 2 | 4 |

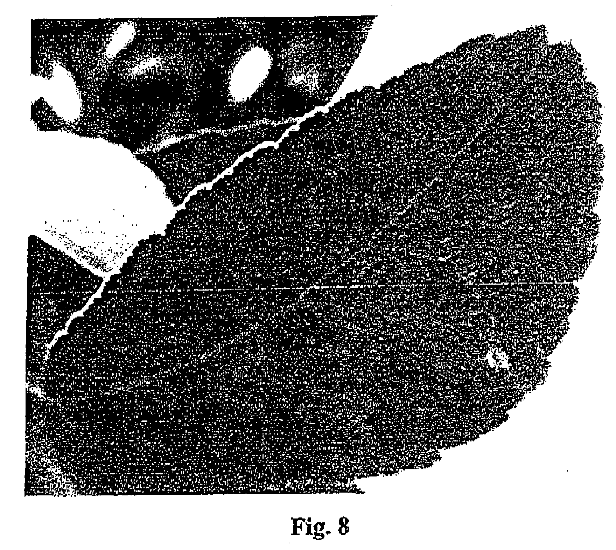

The results clearly indicate the superior adherence property of the CuSiNG suspension over Kocide® 3000. As expected, CuSiNG showed more uniform coverage on the leaf surface. Interestingly, the CuSiNG application to a lemon tree leaf withstood very well the heavy rain fall and thunderstorm that was experienced continuously for five days and is shown in Fig. 8; an enlarged image of a lemon tree leaf with a marbled and visually discernable protective covering of CuSiNG. Most of the Kocide® 3000 formulation washed away after a couple of minutes of rain water spray.

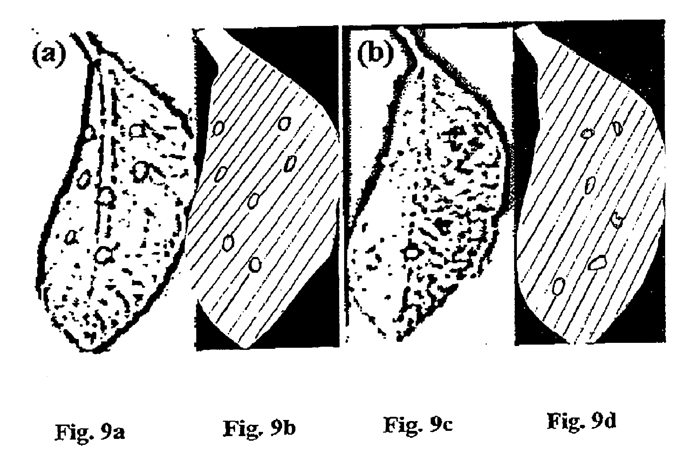

To further evaluate the adherence property of CuSiNG, a fluorescence technique was used. Lemon tree leaves were tagged with fluorescein isothiocyanate (FITC), a green emitting fluorescent dye. The dye covalently attached to the silica. The purpose of fluorescence tagging was to monitor the SiNG adherence property more sensitively using fluorescence measurements. An array of spots was created on the experimental leaf shown in Fig. 9a with fluorescent SiNG material and allowed to dry completely under a dark environment to avoid photobleaching of FITC dyes.

Using a handheld UV light source (366 nm multiband excitation), green fluorescence spots from the leaf surface were clearly noticed before the rain water spraying. Fig. 9b is a drawing of the fluorescent image showing the fluorescent CuSiNG spots in the same pattern as the leaf in Fig. 9a.

After approximately 3 hours, natural rain water was sprayed for about 5 minutes onto the leaf in Fig. 9b, the spray was allowed to dry completely again in the dark and the dry leaf is shown in Fig. 9c, with several CuSiNG spots on the surface. Using a handheld UV light source (366 nm multiband excitation), green fluorescence spots from the leaf surface were clearly noticed *after* the rain water spraying. Fig. 9d is a drawing of the fluorescent image showing the fluorescent CuSiNG material still adhering to the leaf in Fig. 9c in a similar pattern as shown on the leaf in Fig. 9c. The fluorescent images in Fig.s 9b and 9d confirm that the CuSiNG material is not washed off the leaf when subjected to rain water spraying.

The huge surface area of the nanogel (NG) materials is indeed a contributing factor towards releasing adequate amount of "soluble" Cu. The silica-nanogel (SiNG) matrix is porous and the surface is highly hydrated, as disclosed by S. Santra et al. in "Fluorescence Lifetime Measurements to Determine the Core-Shell Nanostructure of FITC-doped Silica Nanoparticles: An Optical Approach to Evaluate Nanoparticle Photostability," Journal of Luminescence, 2006. 117(1) pages 75-82. This silica matrix provides a unique environment, allowing the embedding of various metallic ions, as disclosed in U.S. Patent 6,548,264 to Tan, et al, as well as molecules without a significant change in physical and chemical properties. Characterization studies clearly show that Cu ions are successfully embedded within silica matrix and the embedded Cu ions are released in a sustained manner.

The adherence and slow, sustained release of Cu ions provides several other benefits for the present invention. First, when applied to plants, trees and the like, the leaves and/or branches that fall to the ground provide copper, a highly desired nutrient for the soil and in very low levels that are not environmentally harmful. Also, when the plants and trees are harvested or removed from agricultural production, the treated vegetation can be used for mulch or composting and provide additional Cu nutrients as a fertilizer.

New data, performance and formulations of Cu based nanogel biocides are provided. The new generation of Cu based nanogel biocides have improved Cu bioavailability, a Cu environment in the CuSiNG material that is unique and manipulatable, a Cu based composition with improved adherence properties and properties that permit monitoring to determine whether the biocide needs to be reapplied.

It has been recently discovered that the silica matrix of the silica nanogel is capable of simultaneously hosting two active forms of copper, Cu⁺(I) and Cu⁺⁺(II). Cu (I) is present as Cu₂O in nanocrystalline form (crystal size 1 - 3 nanometers (nm)) and Cu (II) is present as amorphous form (weakly chelated by the silanol groups). The equilibrium established by the two active forms of copper affects the overall Cu ion release kinetics, thus silica nanogel (SiNG) is able to host Cu both in +1 (cuprous, crystalline form) and +2 (cupric, amorphous form) in the same matrix which contributes to sustained Cu release and improved Cu bioavailability. Unlike any existing Cu compounds, the Cu environment in CuSiNG is unique and allows manipulation to improve performance of the biocide composition.

Another recent discovery is the property of the Cu loaded silica nanogel (CuSiNG) material to slowly diffuse out from the location of application in moist environments. Thus, in spray applications, CuSiNG diffuses out and covers newly exposed tissue surfaces of rapidly growing young fruits and leaves, thus minimizing the number of applications per growing season and increasing the longevity and plant surface coverage.

### A New Generation of CuSiNG.

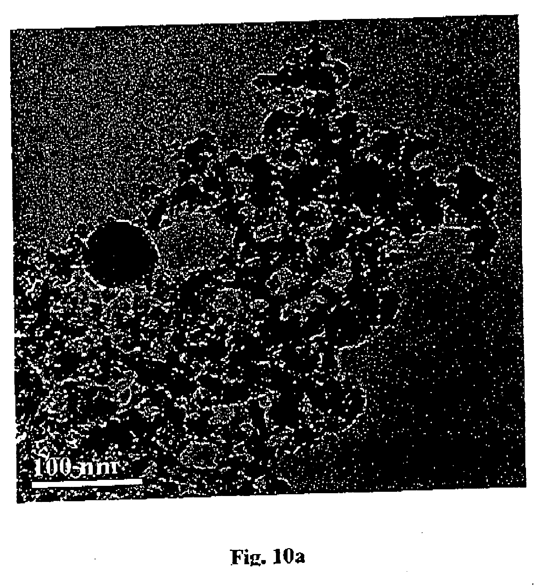

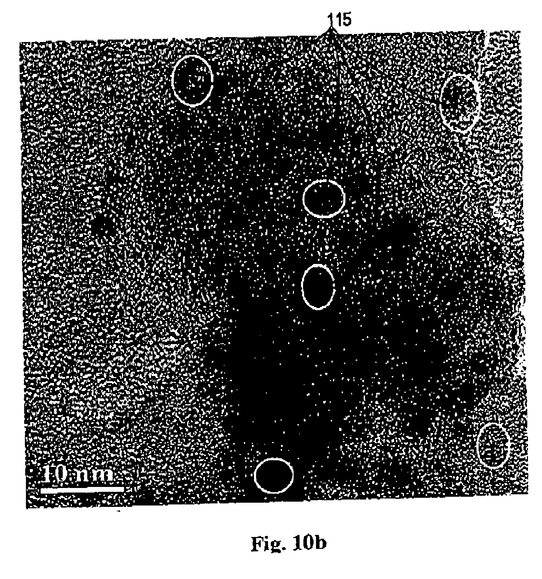

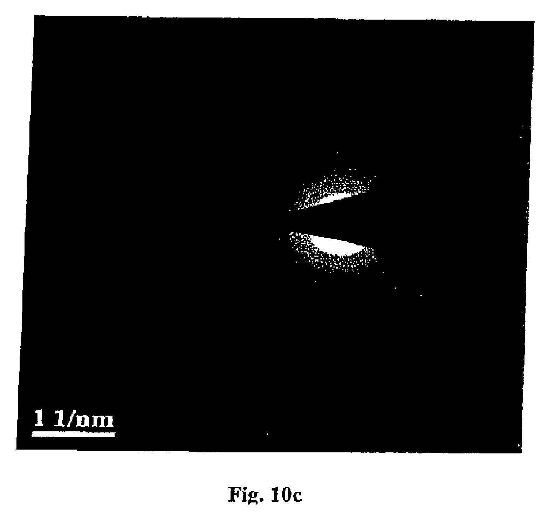

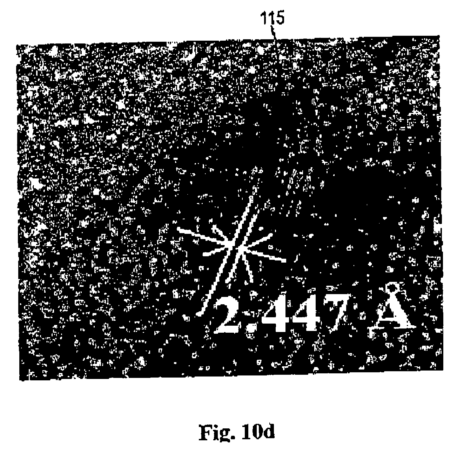

As described in Example 1 above, the gelation of sol particles takes place over time. The gelation process brings together ultra-small (<10 nm size) SiNPs with a further condensation process in the presence of Cu ions, thus forming CuSiNG material, having a network-like structure as shown in **Fig. 10a****.** The reaction medium composition, as well as pH of the medium, favors the formation of 2-3 nm size highly crystalline Cu(I) oxide nanoparticles **115** which remain embedded in silica nanogel matrix as shown in **Fig. 10b** and **Fig. 10d****.** The selected-area electron diffraction (SAED) pattern in **Fig. 10c** indicates the formation of amorphous CuSiNG. **Fig. 10d** is a high resolution transmission electron microscopy (HRTEM) image showing the formation of 1-3 nm dark contrast particles **115,** marked by the parallel lines between two opposing arrows, as crystallites embedded in the amorphous silica matrix. Lattice spacing measured from the enlarged HRTEM image is approximately 2.447A which indicates the formation of Cu₂O crystallites.

The silica nanogel structure of the present invention is loaded with two valence states of Cu which remain intact. The ratio of crystalline to amorphous Cu is controlled by changing the composition of the reaction medium. This provides an excellent opportunity to control Cu release kinetics. Amorphous copper is released quickly and crystalline copper releases slowly. For long-term prevention of citrus canker disease, it is desirable that the Cu based formulation supply Cu ions in a sustained manner without compromising antibacterial efficacy, such as, Cu bioavailability over time.

A hybrid Cu-loaded silica nanogel (H-CuSiNG) has been synthesized wherein the SiNG matrix is loaded with a silane compound to achieve uniform plant surface coverage. A highly dispersed CuSiNG material which includes a silane precursor, such as, 3-(trihydroxysilyl) propyl methylphosphonate (THPMP) is prepared in a one pot, two step procedure as described in Example 1. The process in Example 1 is modified by the addition of the a silane precursor with the copper ions in step one which involves the addition of Cu (II) salts to the reaction medium before the nanoparticle/nanogel synthesis. During the silane nanopargicle/nanogel network formation, copper (Cu) ions will be captured. THPMP is highly water soluble silane containing the negatively charged phosphonate group. In addition to improving dispersibility, the THPMP interacts with Cu ions and modulates the Cu ion release rate, thus improving surface coverage, rain-fastness and longevity of coverage. Silane compounds suitable for the present invention are commercially available from suppliers such as, GELEST, Morrisville, PA 19067.

Detailed materials characterization reveals that the CuSiNG is a unique nanomaterial. Unlike any other existing Cu compounds including Kocide® 3000, the CuSiNG is primarily an amorphous material as confirmed by X-ray diffraction (XRD) study where Cu(II) is complexed with silica silanol groups as confirmed by X-ray photoelectron spectroscopy (XPS) study. HRTEM study confirms that the material embeds 1-3 nm size crystalline Cu(I) oxide nanoparticles **115** that appear as dark contrast areas in **Fig. 10b****.** The BET measurements based on nitrogen adsorption/desorption isotherms confirmed that the CuSiNG material is highly porous with a pore size approximately 7.4 nm and pore volume approximately 1.023 cc/g and possess enormous surface area of approximately 336.1 m²/g.

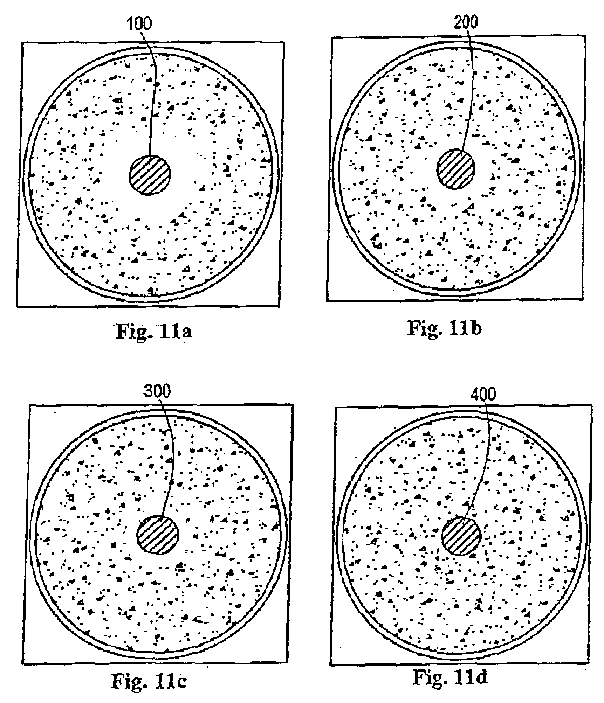

***Antibacterial property of CuSiNG material.*** Assessment of anti-bacterial property of CuSiNG was performed against *X*. *alfalfae* as a model system, using a standard 'disc diffusion assay'. The *X alfalfae* culture grown on nutrient agar plates were incubated with discs containing an equal volume of aqueous solutions of Kocide^{®} 3000, Cu sulfate in deionized (DI) water, CuSiNG at a pH 7 value and SiNG. The metallic Cu content was kept identical in all these Cu based aqueous solutions. After overnight incubation at 30 °C, we found that the CuSiNG **100** in **Fig. 11a** exhibited very significant antimicrobial activity for *X alfalfa.* This was indicated by a marked zone of clearance extending away from the corresponding disc. Notably, the anti-bacterial activity of the CuSiNG was more pronounced than that of Kocide^{®} 3000 **200** in **Fig. 11b** and Cu sulfate **300** in **Fig. 11c** even though the metallic Cu content remained identical. It should be noted that, as expected, SiNG **400** did not show any antibacterial activity as shown in **Fig. 11d****.**

The above experimental results suggest that Cu bioavailability is improved when two valence states of Cu (Cu¹⁺ and Cu²⁺) are loaded in a SiNG matrix. This improved efficacy cannot be explained based on reported solubility data of tested materials. The solubility product (*K*_{SP}) value of Cu-hydroxide is 2.20 x 10⁻²⁰. Slow release of Cu²⁺ ion is therefore expected from Kocide^{®} 3000. Cu-sulfate, on the other hand is a highly water-soluble salt with acidic pH (pH ∼ 3.0) and therefore abundant Cu²⁺ release is expected. Agar, a sugar based material, is known to bind to metal ions. It is therefore expected that it will bind to Cu ions and retard Cu diffusion irrespective of the tested Cu containing materials such as Cu sulfate, Kocide^{®} 3000 and CuSiNG materials. Furthermore, CuSiNG materials should have less soluble Cu in comparison to Cu-sulfate and more soluble Cu in comparison to Kocide® 3000. Therefore, the unexpectedly high anti-bacterial activity of CuSiNG material is possibly due to its ability to migrate, via diffusion, from the disc and interact directly with the bacteria. This direct interaction will increase the local Cu concentration on the bacterial cell surface, thus effectively killing them.

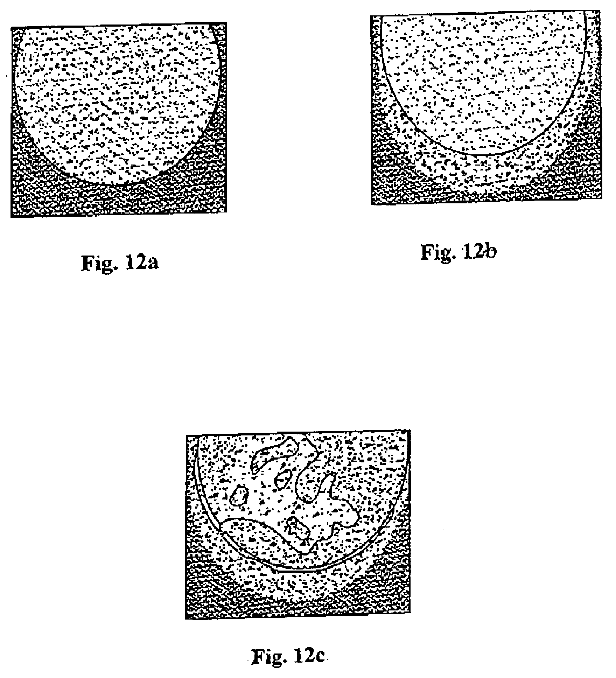

***Diffusion of CuSiNG material.*** To test the diffusion properties of CuSiNG, fluorescent SiNG was loaded with a metallorganic orange-red emitting fluorescent dye, tris(2,2'-bipyridyl)dichlororuthenium(II) hexahydrate (Rubpy), a highly water-soluble dye with excitation at 455 nm wave length and emission 600 nm wave length. The fluorescently bright and photostable dye added to SiNG material is hereafter referred to as RuSiNG. The RuSiNG was washed in deionized (DI) water several times to ensure removal of any unbound Rubpy. A qualitative test was developed for the assessment of Rubpy loaded SiNG (RuSiNG) diffusion property using a nutrient agar plate as substrate. In a typical procedure, 200 µL of RuSiNG (∼ 1 mg/mL) was administered to a 13 mm blank paper disc. The disc was then placed in the center of a nutrient agar plate. The plate was refrigerated for a week prior to imaging under a fluorescence microscope (Olympus MVX10 equipped with DP72 color camera; filter set: 380-420 nm bandpass excitation filter and 475 nm longpass emission filter). It was clearly seen that the RuSiNG diffused out of the disc as shown in **Figures 12a-12c****.** In **Fig. 12a** there is a fluorescent image of a blank disc on a nutrient agar plate. **Fig. 12b****,** shows a fluorescent image of a RuSiNG treated disc on a nutrient agar plate after refrigeration for 7 days. **Fig. 12c** is a fluorescent image of the same agar plate in **Fig. 12b** with the RuSiNG disc removed. This diffusion study shows that SiNG material was able to diffuse on an agar substrate under refrigerated condition.

In summary, the diffusion study indicates that the CuSiNG material of the present invention is capable of diffusing in a moist environment (e.g., typical humid Florida weather) over time. This diffusion property is critical for protecting rapidly expanding young citrus fruits and leaves from the canker threat. Once applied in the beginning of the season, the CuSiNG material slowly spreads out over time to cover newly formed tissue surface, thus leaving no room for canker bacteria to cause infection.

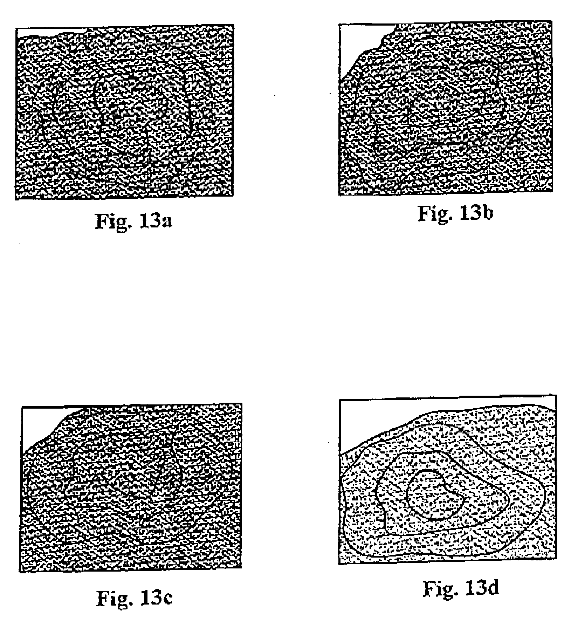

***Assessment of adherence property of CuSiNG material.*** It was visually observed that CuSiNG material has better adherence property (determined upon spray application of rain water continuously for 5 minutes) in comparison to Kocide^{®} 3000 when studied on a lemon plant. To further assess the adherence property of the SiNG material at the microscopic levels, we used fluorescent RuSiNG as a model material. A small aliquot (10 µL) of RuSiNG (~ 1 mg/mL) was delivered as a spot on a fresh, clean citrus leaf and allowed to dry. The leaf was viewed under a fluorescence microscope (Olympus MVX10 microscope with long working distance) after the initial administration and following 3 subsequent washes. Each wash was performed by holding the leaf under a fast-running faucet for a set period of time. A series of fluorescence images ware taken for pre-wash **(****Fig. 13a**), after 1 min wash **(****Fig. 13b****),** after 6 min wash **(****Fig. 13c****)** and after 16 min **(****Fig. 13d****)** wash samples. Images clearly show that the SiNG material has an extremely strong adherence property to the citrus leaf surface. The strong adherence property of the SiNG material reflects strong van der Waals interaction (intermolecular forces) between the CuSiNG and the leaf surface. Such strong adhesion of SiNG material is due to its nanoscale properties, such as, high surface area and network like structure. A study of CuSiNG adherence properties clearly demonstrates the role of nanotechnology in drastically improving the adherence property of SiNG based material to a citrus leaf surface.

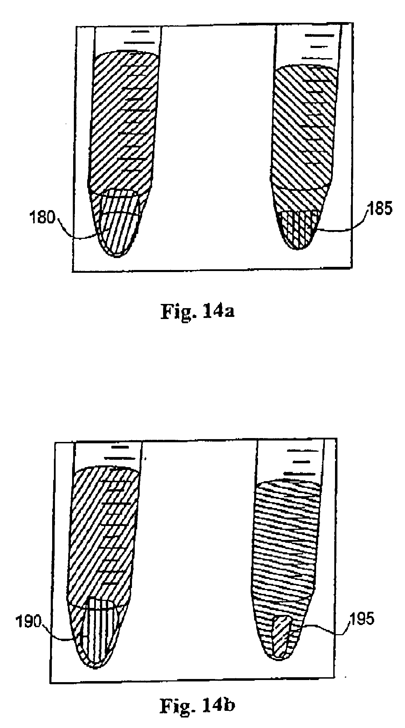

***Fluorescence to measure need for repeated applications of CuSiNG.*** A fluorescence based sensing technique allows monitoring of Cu release in real-time. The sensing mechanism is based on fluorescence quenching. It is well known that Cu ions can effectively quench fluorescence of fluorescent dyes through energy- and/or electron-transfer processes. Therefore, if a fluorescent dye, such as, Rubpy is incorporated into CuSiNG material, fluorescence from Rubpy dye will be quenched. Upon release of Cu ions, fluorescence of Rubpy will be restored. The feasibility of this mechanism, was tested when Rubpy loaded CuSiNG material (RuCuSiNG) was synthesized by simple mixing. As expected, fluorescence from the Rubpy was completely quenched due to the presence of Cu ions. The fluorescence was however restored completely when Cu was removed by treating RuCuSiNG with ethylenediaminetetraacetic acid (EDTA), a strong chelating agent as shown in **Figures 14a and 14b****.** In **Fig 14a** centrifuged RuCuSiNG material is shown as a heavy deposit **180** in the bottom of the tube on the left and in a dispersed phase **185** in the tube on the right when treated with EDTA there is evidence of fluorescence under room light. In **Fig. 14b** the centrifuged RuCuSiNG material **190** on the left is treated with EDTA, a chelating agent that removes copper from the solution and under a hand-held 366nm UV wavelength excitation light, bright fluorescence **195** is shown in the tube on the right. This occurs after the Cu ion is extracted from the material as Cu-EDTA complex. This provides a basis of using a fluorescence based highly-sensitive sensing technique to monitor Cu release from the material in real time.

Due to high sensitivity of fluorescence technique and the large signal-to-noise ratio, sensitive detection of Cu release is anticipated. Based on this sensing study, we will be able to predict Cu bioavailability and screen a series of CuSiNG materials with increasing ratio of amorphous (Cu²⁺) to crystalline (Cu¹⁺) forms.

Another benefit of the Cu-loaded silica nanoparticles and nanogel of the present invention involves controlling mold and mildew growth. The copper (Cu) loaded silica nanoparticle/nanogel formulations of the present invention have superior antibacterial/ antifungal activity for treating areas that favor and foster the growth of mold and mildew; in addition, a single treatment remains effective for from at least two months to approximately six months because the release of ionic Cu occurs in a slow, sustained manner and in quantities that inhibit germination of fungal spores, the primary "seeds" responsible for dissemination and reproduction of the fungus or bacterium. Because the spore or cell removed from the current infection cycle does not mature nor reproduce in the presence of copper, the fungus or bacterium is effectively killed. Bathrooms, kitchens, interior and exterior areas of structures subject to warm, moist conditions remain free of unsightly mold and mildew when treated with the silica-based nanoformulation of the present invention.

In conclusion, for the first time, a sustained Cu release mechanism is provided using a uniquely designed copper (Cu) containing silica nanoparticle/nanogel (SiNP/NG) formulation. The silica-based nanoformulation finds immediate application for citrus canker treatment. Additional uses of antibacterial/antifungal compositions containing SiNP/NG are for general purpose antibacterial/antifungal agents in agriculture and horticulture, including, but not limited to use on vegetables, flowers, grass, other plants, household applications, fabrics, leathers, plastics, paints and the like. The nanotechnology-enabled CuSiNG design is a "revolutionary re-invention" of Cu for safe application in modem agriculture in the 21^{st} century.

## Claims

1. A method for synthesizing a silica-based nanoformulation containing antibacterial and antifungal agents using a one-pot, acid-catalyzed, sol-gel process, comprising the steps of:
selecting a reaction vessel containing an acidic reaction medium;
adding tetraethoxysilane (TEOS), water, ethanol and an antibacterial or antifungal agent containing copper ions to the reaction vessel with the acidic medium to form mixture I;
allowing the reaction of mixture I to continue for approximately one hour to facilitate the formation of a plurality of silica-based nanoparticles;
adding a neutralizing agent after the formation of a plurality of nanoparticles to adjust the pH value of the reaction mixture to approximately 7.0;
allowing the neutralized reaction of mixture I to continue for approximately 12 hours, preferably overnight, to allow gelation to occur, and form a nanogel structure consisting of interconnected nanoparticles; and
collecting a porous silica-based nanogel having antibacterial and antifungal agents bound in a plurality of interconnected nanoparticles of the nanogel;
wherein antibacterial or antifungal agent is a plurality of copper (Cu) ions sourced from a copper compound;
**characterized in that** the plurality of interconnected nanoparticles of the nanogel contain
- a plurality of Cu ions electrostatically bound to the nanoparticle core,
- a plurality of embedded Cu nanocrystallites including at least Cu₂O,
- a plurality of Cu ions covalently bound to a silica matrix with a hydrated shell surface, nanopores of the nanoparticle and porous channels of nanogel, and
- a plurality of Cu oxide/hydroxide as nanoclusters/nanoparticles bound to the surface of the nanoparticle.

2. A composition for treating and preventing disease in a plant species comprising a silica-based nanoformulation comprising a plurality of copper ions embedded in a plurality of silica nanoparticles or comprising a plurality of copper ions embedded in a silica nanogel, wherein the silica-based nanoformulation includes a silica matrix in a copper ion loaded silica nanogel (CuSiNG); and
**characterized in that** the silica matrix in the copper ion loaded silica nanogel (CuSiNG) simultaneously hosts two active forms of copper wherein a first form of copper is amorphous and a second form of copper is crystalline and the equilibrium established by the two active forms of copper can be adjusted to control copper ion release kinetics and improve copper ion bioavailability.

3. The composition of claim 2, wherein the source of the plurality of copper ions is from copper compounds selected from the group consisting of metallic copper, copper oxide, copper oxychloride, copper sulfate, copper hydroxide and mixtures thereof.

4. The composition of claim 2, further including a silane precursor compound to achieve uniform plant surface coverage, modulate copper ion release rate, improve rain-fastness and increase longevity of plant surface coverage.

5. The composition of claim 4, wherein the silane precursor compound is 3-(trihydroxysilyl) propyl methylphosphonate (THPMP).

## Patentansprüche

1. Ein Verfahren zum Synthetisieren einer silikatischen Nanoformulierung, die antibakterielle und antimykotische Mittel enthält, unter Anwendung eines Säure-katalysierten Sol-Gel-Prozesses in einem Topf, mit den Schritten:
Auswählen eines Reaktionsbehälters, der ein saures Reaktionsmedium enthält;
Zugeben von Tetraethoxysilan (TEOS), Wasser, Ethanol und eines antibakteriellen oder antimykotischen, Kupferionen enthaltenden Mittels in den Reaktionsbehälter mit dem sauren Medium, um eine Mischung I auszubilden;
es der Reaktion der Mischung I Erlauben, für ungefähr eine Stunde fortzufahren, um die Bildung einer Vielzahl von silikatischen Nanopartikeln zu erleichtern;
Hinzugeben eines neutralisierenden Mittels nach der Bildung einer Vielzahl von Nanopartikeln, um den PH-Wert der Reaktionsmischung auf ungefähr 7,0 einzustellen;
es der neutralisierten Reaktion der Mischung I Erlauben, für ungefähr 12 Stunden fortzufahren, vorzugsweise über Nacht, um eine Gelbildung zu erlauben, und Ausbilden einer Nanogelstruktur bestehend aus untereinander verbundenen Nanopartikeln; und
Aufnehmen eines porösen silikatischen Nanogels mit antibakteriellen und antimykotischen Mitteln gebunden in einer Vielzahl von miteinander verbundenen Nanopartikeln des Nanogels;
wobei das antibakterielle oder antimykotische Mittel eine Vielzahl von Kupfer (Cu)-Ionen aus der Quelle einer Kupferverbindung ist;
**dadurch gekennzeichnet, dass** die Vielzahl der untereinander verbundenen Nanopartikel des Nanogels enthält
- eine Vielzahl von Cu-Ionen, die elektrostatisch an den Nanopartikelkern gebunden sind,
- eine Vielzahl von eingebetteten Cu-Nanokristalliten, die zumindest Cu₂O enthalten,
- eine Vielzahl von Cu-Ionen, die kovalent an eine silikatische Matrix mit einer hydratisierten Hüllenoberfläche, Nanoporen der Nanopartikel und poröse Kanäle des Nanogels gebunden sind, und
- eine Vielzahl von Cu-Oxiden/-Hydroxiden als Nanocluster/Nanopartikel gebunden an die Oberfläche der Nanopartikel.

2. Eine Zusammensetzung zur Behandlung und Verhinderung von Krankheit bei einer Pflanzenart mit einer silikatischen Nanoformulierung mit einer Mehrzahl von Kupferionen eingebettet in eine Vielzahl von silikatischen Nanopartikeln oder mit einer Mehrzahl von Kupferionen eingebettet in ein silikatisches Nanogel, wobei die silikatische Nanoformulierung eine silikatische Matrix in einem Kupferionen-beladenen silikatischen Nanogel (CuSiNG) umfasst; und
**dadurch gekennzeichnet, dass** die silikatische Matrix in dem Kupferionen-beladenen silikatischen Nanogel (CuSiNG) gleichzeitig zwei aktive Kupferformen aufnimmt, wobei eine erste Kupferform amorph ist und eine zweite Kupferform kristallin ist und das Gleichgewicht, das zwischen den beiden aktiven Kupferformen besteht, eingestellt werden kann, um die Kupferionenfreisetzungskinetik zu steuern und die Kupferionenbioverfügbarkeit zu verbessern.

3. Die Zusammensetzung nach Anspruch 2, wobei die Quelle der Vielzahl von Kupferionen eine Kupferverbindung ist, die aus der Gruppe ausgewählt ist, welche besteht aus metallischem Kupfer, Kupferoxid, Kupferoxidchlorid, Kupfersulfat, Kupferhydroxid und Mischungen davon

4. Die Zusammensetzung nach Anspruch 2, die weiterhin eine Silanvorformverbindung umfasst, um eine gleichmäßige Pflanzenoberflächenabdeckung zu erreichen, die Kupferionenfreisetzungsrate zu modulieren, die Regenfestigkeit zu verbessern und die Langlebigkeit der Pflanzenoberflächenabdeckung zu erhöhen.

5. Die Zusammensetzung nach Anspruch 4, wobei die Silanvorformverbindung 3-(Trihydroxylsilyl)propylmethylphosphonat (THPMP) ist.

## Revendications

1. Procédé de synthèse d'une nanoformulation à base de silice contenant des agents antibactériens et antifongiques à l'aide d'un procédé sol-gel monotope, catalysé par un acide, comprenant les étapes suivantes:
choix d'un récipient à réaction contenant un milieu réactionnel acide;
ajout de tétraéthoxysilane (TEOS), d'eau, d'éthanol et d'un agent antibactérien ou antifongique contenant des ions de cuivre au récipient à réaction avec le milieu acide pour former le mélange I;
poursuite de la réaction du mélange I pendant environ une heure pour faciliter la formation d'une pluralité de nanoparticules à base de silice;
ajout d'un agent de neutralisation après la formation d'une pluralité de nanoparticules pour ajuster la valeur de pH à l'aide du mélange réactionnel à environ 7,0;
poursuite de la réaction neutralisée du mélange I pendant environ 12 heures, de préférence pendant une nuit, pour laisser la gélification se produire, et pour former une structure de nanogel constituée de nanoparticules interconnectées; et
collecte d'un nanogel poreux à base de silice comportant des agents antibactériens et antifongiques liés dans une pluralité de nanoparticules interconnectées du nanogel;
dans lequel l'agent antibactérien ou antifongique est une pluralité d'ions de cuivre (Cu) provenant d'un composé à base de cuivre;
**caractérisé en ce que** la pluralité de nanoparticules interconnectées du nanogel contiennent
- une pluralité d'ions de Cu électrostatiquement liés au coeur des nanoparticules,
- une pluralité de nanocristallites de Cu incorporés incluant au moins du Cu₂O,
- une pluralité d'ions de Cu liés par covalence à une matrice de silice présentant une surface constituée d'une enveloppe hydratée, des nanopores de la nanoparticule et des canaux poreux de nanogel, et
- une pluralité d'oxyde/hydroxyde de Cu en tant que nanoagrégats/nanoparticules liés à la surface de la nanoparticule.

2. Composition destinée au traitement et à la prévention d'une maladie d'une espèce végétale comprenant une nanoformulation à base de silice comprenant une pluralité d'ions de cuivre incorporés dans une pluralité de nanoparticules de silice ou comprenant une pluralité d'ions de cuivre incorporés dans un nanogel de silice, la nanoformulation à base de silice incluant une matrice de silice dans un nanogel de silice chargé d'ions de cuivre (CuSiNG); et
**caractérisée en ce que** la matrice de silice du nanogel de silice chargé d'ions de cuivre (CuSiNG) accueille simultanément deux formes actives de cuivre dans lesquelles une première forme de cuivre est amorphe et une deuxième forme de cuivre est cristalline et l'équilibre établi par les deux formes actives de cuivre peut être ajusté pour réguler la cinétique de libération des ions de cuivre et améliorer la biodisponibilité des ions de cuivre.

3. Composition selon la revendication 2, dans laquelle la source de la pluralité d'ions de cuivre provient de composés à base de cuivre choisis dans le groupe constitué du cuivre métallique, de l'oxyde de cuivre, de l'oxychlorure de cuivre, du sulfate de cuivre, de l'hydroxyde de cuivre et des mélanges de ceux-ci.

4. Composition selon la revendication 2, incluant en outre un composé précurseur de silane permettant d'obtenir une couverture uniforme de la surface des plantes, de moduler le taux de libération des ions de cuivre, d'améliorer la résistance à la pluie et d'augmenter la longévité de la couverture de la surface des plantes.

5. Composition selon la revendication 4, dans laquelle le composé précurseur de silane est le méthylphosphonate de 3-(trihydroxysilyl)propyle (THPMP).
